(19) Europäisches Patentamt
European Patent Office
Office européen des brevets

(11) Veröffentlichungsnummer: **0 126 361**
**A1**

# (12) EUROPÄISCHE PATENTANMELDUNG

(21) Anmeldenummer: **84105088.3**

(22) Anmeldetag: **05.05.84**

(51) Int. Cl.³: **C 07 D 213/64**, C 07 C 69/712, C 07 C 67/14, C 07 C 67/10, C 07 C 67/00, C 07 C 67/31, A 01 N 43/40, A 01 N 37/38

(30) Priorität: **20.05.83 DE 3318354**

(43) Veröffentlichungstag der Anmeldung: **28.11.84 Patentblatt 84/48**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**

## (54) Optisch aktive Propionsäure-Derivate.

(57) Die Erfindung betrifft neue optisch aktive Propionsäure-Derivate der Formel

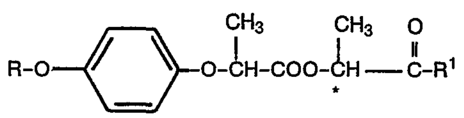

(I)

in welcher
R für die Reste der Formeln

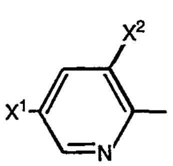

oder

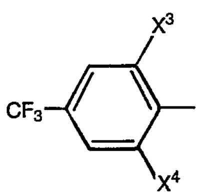

steht, worin

$X^1$ für Halogen oder Trifluormethyl steht,
$X^2$ für Wasserstoff, Halogen oder Trifluormethyl steht und
$X^3$ und $X^4$ unabhängig voneinander für Wasserstoff oder Halogen stehen,
und
$R^1$ für Alkoxy oder Alkylthio steht,
mehrere Verfahren zur Herstellung dieser neuen Stoffe und deren Verwendung als Herbizide.

EP 0 126 361 A1

ACTORUM AG

BAYER AKTIENGESELLSCHAFT
Konzernverwaltung RP
Patentabteilung

5090 Leverkusen, Bayerwerk
Dü/bo/c
Ib

## Optisch aktive Propionsäure-Derivate

Die Erfindung betrifft neue optisch aktive Propionsäure-Derivate, mehrere Verfahren zu deren Herstellung und deren Verwendung als Herbizide.

Es ist bereits bekannt geworden, daß bestimmte optisch aktive Phenoxypropionsäure-Derivate herbizide Eigenschaften besitzen (vgl. DE-OS 27 58 002). So kann zum Beispiel das R-Enantiomere des 2-/4-(3,5-Dichlor-pyridyl-2-oxy)-phenoxy/-propionsäure-ethylesters zur Unkrautbekämpfung eingesetzt werden. Die Wirkung dieses Stoffes ist gut, läßt aber bei niedrigen Aufwandmengen manchmal zu wünschen übrig.

Es wurden nun neue optisch aktive Propionsäure-Derivate der Formel

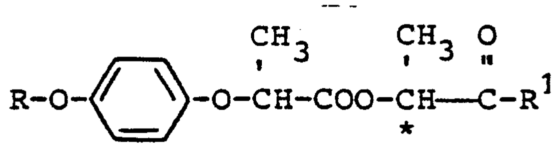

(I)

in welcher

R für die Reste der Formeln

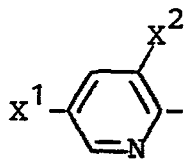 oder 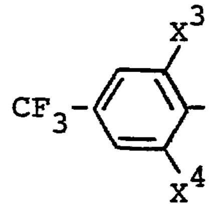 steht,

worin

$X^1$ für Halogen oder Trifluormethyl steht,

$X^2$ für Wasserstoff, Halogen oder Trifluormethyl steht,

$X^3$ für Wasserstoff oder Halogen steht und

$X^4$ für Wasserstoff oder Halogen steht,

und

$R^1$ für Alkoxy oder Alkylthio steht,

gefunden.

In der Formel (I) ist das Asymmetriezentrum durch ein (*) gekennzeichnet. Die Erfindung betrifft sowohl diejenigen Stoffe der Formel (I), in denen das asymmetrisch substituierte Kohlenstoffatom die R-Konfiguration aufweist, als auch diejenigen, in denen das asymmetrisch substituierte Kohlenstoffatom die S-Konfiguration aufweist.

Weiterhin wurde gefunden, daß man die neuen optisch aktiven Propionsäure-Derivate der Formel (I) erhält, wenn man

a) Phenoxycarbonsäurechloride der Formel

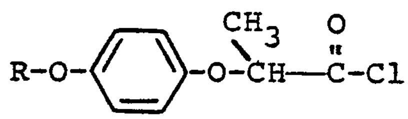 (II)

in welcher

R die oben angegebene Bedeutung hat,

mit optisch aktiven Milchsäure-Derivaten der Formel

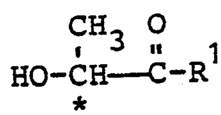 (III)

in welcher

$R^1$ die oben angegebene Bedeutung hat,

gegebenenfalls in Gegenwart eines Säureakzeptors sowie gegebenenfalls in Gegenwart eines Verdünnungsmittels umsetzt,

oder

b) Phenol-Derivate der Formel

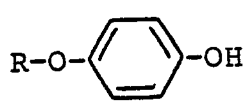 (IV)

in welcher

R die oben angegebene Bedeutung hat,

mit einem optisch aktiven Carbonsäure-Derivat der Formel

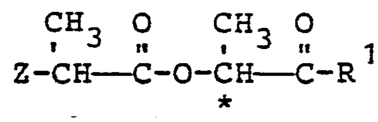

(V)

in welcher

$R^1$ die oben angegebene Bedeutung hat und

Z für Chlor, Brom, Tosylat oder Mesylat steht,

gegebenenfalls in Gegenwart eines Säureakzeptors sowie gegebenenfalls in Gegenwart eines Verdünnungsmittels umsetzt,

oder

c) Phenoxycarbonsäure-Salze der Formel

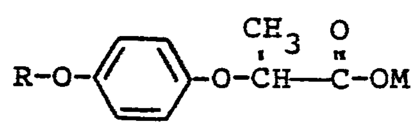

(VI)

in welcher

R die oben angegebenen Bedeutung hat und

M für ein Alkalimetall-Kation oder für ein Äquivalent eines Erdalkalimetall-Kations steht,

mit optisch aktiven Halogenverbindungen der Formel

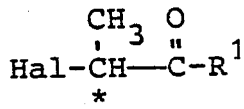

(VII)

in welcher

$R^1$ die oben angegebene Bedeutung hat und

Hal für Chlor oder Brom steht,

gegebenenfalls in Gegenwart eines Säureakzeptors sowie gegebenenfalls in Gegenwart eines Verdünnungsmittels umsetzt.

Schließlich wurde gefunden, daß sich die neuen optisch aktiven Propionsäure-Derivate der Formel (I) durch eine hervorragende herbizide Wirksamkeit auszeichnen.

Überraschenderweise besitzen die erfindungsgemäßen optisch aktiven Propionsäure-Derivate der Formel (I) bessere herbizide Eigenschaften als die konstitutionell ähnlichsten vorbekannten Wirkstoffe.

Die erfindungsgemäßen Verbindungen sind durch die Formel (I) allgemein definiert. In dieser Formel steht R für die Reste

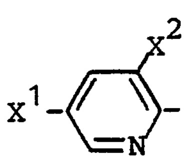 oder 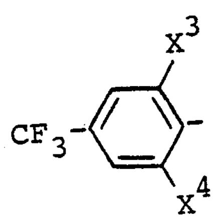 , in welchen

$X^1$ vorzugsweise für Chlor oder Trifluormethyl steht und $X^2$ vorzugsweise für Wasserstoff oder Chlor steht, wobei $X^2$ insbesondere dann für Wasserstoff steht, wenn $X^1$ für Trifluormethyl steht, und $X^2$ insbesondere dann für Chlor steht, wenn auch $X^1$ für Chlor steht, und in welchen ferner $X^3$ vorzugsweise für Wasserstoff oder Chlor steht und $X^4$ vorzugsweise für Wasserstoff oder Chlor steht. $R^1$ steht vorzugsweise für Alkoxy mit 1 bis 4 Kohlenstoffatomen oder Alkylthio mit 1 bis 4 Kohlenstoffatomen.

Eine besonders bevorzugte Gruppe erfindungsgemäßer Verbindungen sind diejenigen Stoffe der Formel (I), in denen der Rest $R^1$ die oben angegebenen vorzugsweisen Bedeutungen hat und das asymmetrisch substituierte Kohlenstoffatom im Esterteil die S-Konfiguration aufweist.

Eine andere besonders bevorzugte Gruppe erfindungsgemäßer Verbindungen sind diejenigen Stoffe der Formel (I), in denen der Rest $R^1$ die oben angegebenen vorzugsweisen Bedeutungen hat und das asymmetrisch substituierte Kohlenstoffatom im Esterteil die R-Konfiguration aufweist.

Verwendet man 2-/4-(4-Trifluormethyl-phenoxy)-phenoxy/-propionsäurechlorid und das S-Enantiomere des Milchsäure-ethylesters als Ausgangsstoffe, so kann der Verlauf des erfindungsgemäßen Verfahrens (a) durch das folgende Formelschema wiedergegeben werden:

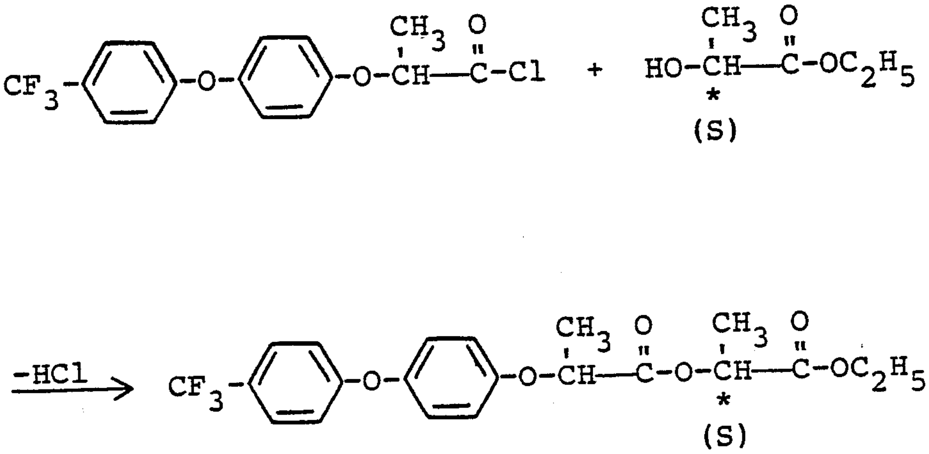


Verwendet man 4-(2-Chlor-4-trifluormethyl-phenoxy)-phenol und das S-Enantiomere des 2-Tosyloxy-propionsäure-(1-ethoxycarbonyl)-ethylesters als Ausgangsstoffe, so kann der Verlauf des erfindungsgemäßen Verfahrens (b) durch das folgende Formelschema wiedergegeben werden:

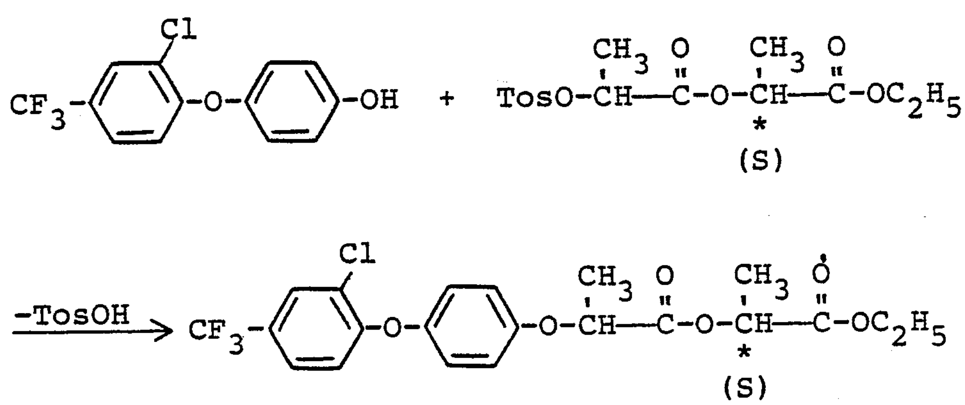


Tos = 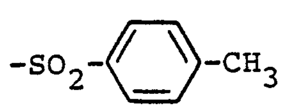 (= Tosyl)

Verwendet man 2-/4-(5-Trifluormethyl-pyridyl-2-oxy)-phenoxy/-propionsäure-natriumsalz und das R-Enantiomere des 2-Brom-propionsäure-ethylesters als Ausgangsstoffe, so kann der Verlauf des erfindungsgemäßen Verfahrens (c) durch das folgende Formelschema wiedergegeben werden:

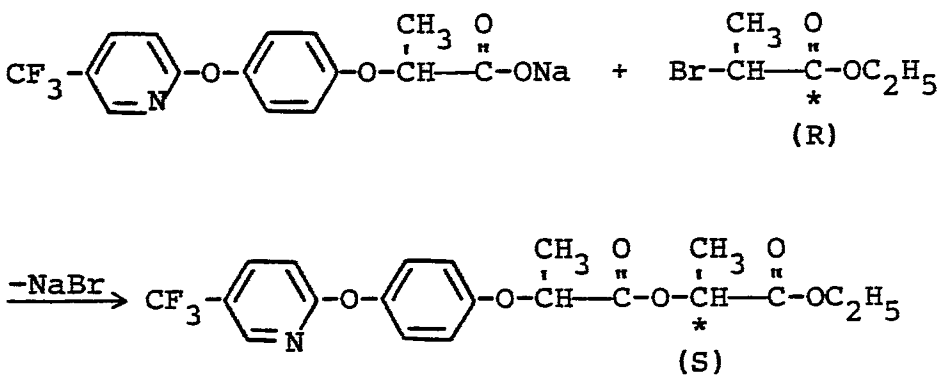


Die bei dem erfindungsgemäßen Verfahren (a) als Ausgangsstoffe benötigten Phenoxycarbonsäurechloride sind durch die Formel (II) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden.

Die Phenoxycarbonsäurechloride der Formel (II) sind bekannt oder lassen sich nach bekannten Methoden herstellen (vgl. DE-OS 26 28 384, DE-OS 28 12 571 und US-PS 4 046 553). So kann man die Stoffe der Formel (II) zum Beispiel dadurch erhalten, daß man die zugrundeliegenden Säuren mit Thionylchlorid umsetzt.

Die bei dem erfindungsgemäßen Verfahren (a) weiterhin als Ausgangsstoffe benötigten optisch aktiven Milchsäure-Derivate sind durch die Formel (III) allgemein definiert. In dieser Formel hat $R^1$ vorzugsweise diejenigen Bedeutungen, die bereits im Zusammenhang mit der Beschreibung der erfindungsgemäßen Stoffe vorzugsweise für diesen Substituenten genannt wurden.

Die optisch aktiven Milchsäure-Derivate der Formel (III) sind bekannt oder lassen sich nach bekannten Methoden der Racemat-Spaltung herstellen (vgl. DE-OS 27 58 002 und DE-OS 29 06 087).

Verwendet man bei dem erfindungsgemäßen Verfahren (a) die R-Enantiomeren der Milchsäure-Derivate der Formel (III), so entstehen diejenigen erfindungsgemäßen Stoffe, die am asymmetrisch substituierten Kohlenstoffatom im Esterteil die R-Konfiguration aufweisen. Entsprechend entstehen aus den S-Enantiomeren diejenigen Stoffe, die am asymmetrisch substituierten Kohlenstoffatom im Esterteil die S-Konfiguration aufweisen.

Die bei dem erfindungsgemäßen Verfahren (b) als Ausgangsstoffe benötigten Phenol-Derivate sind durch die Formel (IV) definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Phenol-Derivate der Formel (IV) sind bekannt oder lassen sich nach bekannten Methoden in einfacher Weise herstellen (vgl. DE-OS 2 758 002, DE-OS 2 812 571, EP-OS 483 und EP-OS 1 473).

Die bei dem erfindungsgemäßen Verfahren (b) weiterhin als Ausgangsstoffe benötigten optisch aktiven Carbonsäure-Derivate sind durch die Formel (V) allgemein definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden. Z steht für Chlor, Brom, Tosylat ($-OSO_2-$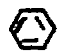$-CH_3$) oder Mesylat ($-OSO_2-CH_3$).

Die optisch aktiven Carbonsäure-Derivate der Formel (V) sind bekannt oder lassen sich nach bekannten Methoden herstellen. So erhält man diese Verbindungen, indem man Säurechloride der Formel

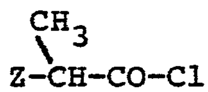

(VIII)

in welcher

Z die oben angegebene Bedeutung hat,

mit optisch aktiven Milchsäure-Derivaten der Formel

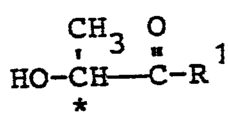

(III)

in welcher

$R^1$ die oben angegebene Bedeutung hat,

gegebenenfalls in Gegenwart eines Säureakzeptors sowie gegebenenfalls in Gegenwart eines Verdünnungsmittels umsetzt.

Bei diesem Verfahren zur Herstellung der optisch aktiven Carbonsäure-Derivate der Formel (V) entsprechen die Umsetzungsbedingungen denen des erfindungsgemäßen Verfahrens (a) (vgl. unten).

Die bei dem erfindungsgemäßen Verfahren (c) als Ausgangsstoffe benötigten Phenoxycarbonsäure-Salze sind durch die Formel (VI) definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden. M steht vorzugsweise für ein Natrium- oder Kalium-Kation, oder für ein Äquivalent eines Calcium-Kations.

Die Phenoxycarbonsäure-Salze der Formel (VI) sind bekannt oder lassen sich in einfacher Weise nach bekannten Methoden herstellen (vgl. Deutsche Offenlegungsschriften 28 02 818, 26 46 124, 26 28 384, 28 12 571 und Europäische Offenlegungsschriften 483 und 1 473).

Die bei dem erfindungsgemäßen Verfahren (c) weiterhin als Ausgangsstoffe benötigten optisch aktiven Halogenverbindungen sind durch die Formel (VII) definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden. Hal steht für Chlor oder Brom.

Die optisch aktiven Halogenverbindungen der Formel (VII) sind bekannt oder lassen sich in einfacher Weise nach bekannten Methoden aus den zugrundeliegenden optisch aktiven Milchsäure-Derivaten herstellen.

Verwendet man bei dem erfindungsgemäßen Verfahren (c) R-Enantiomere der Halogenverbindungen der Formel (VII), so entstehen diejenigen erfindungsgemäßen Stoffe, die am Asymmetriezentrum die S-Konfiguration aufweisen, da im Zuge der Umsetzung eine Walden'sche Umkehr erfolgt. Entsprechend entstehen aus den S-Enantiomeren der Halogenverbindungen der Formel (VII) diejenigen erfindungsgemäßen Stoffe, die am Asymmetriezentrum die R-Konfiguration aufweisen.

Die erfindungsgemäßen Verfahren (a), (b) und (c) zur Herstellung der neuen optisch aktiven Propionsäure-Derivate der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methylisopropyl-, und Methyl-isobutyl-keton, Ester, wie Essigsäure-methylester und -ethylester, Nitrile, wie

z.B. Acetonitril und Propionitril, Amide, wie z.B. Dimethylformamid, Dimethylacetamid und N-Methyl-pyrrolidon sowie Dimethylsulfoxid, Tetramethylensulfon und Hexamethylphosphorsäuretriamid.

Als Säurebindemittel können sowohl bei den erfindungsgemäßen Verfahren (a) und (b) als auch bei dem Verfahren (c) alle üblicherweise für derartige Umsetzungen verwendbaren Säurebindemittel eingesetzt werden. Vorzugsweise in Frage kommen Alkalihydroxide, wie z.B. Natrium- und Kaliumhydroxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diaza-bicyclo-[4.3.0]-nonen-5(DBN) und 1,8-Diaza-bicyclo-[5.4.0]-undec-7-en (DBU).

Die Reaktionstemperaturen können sowohl bei dem erfindungsgemäßen Verfahren (a) als auch bei den Verfahren (b) und (c) innerhalb eines größeren Bereiches variiert werden. Im allgemeinen arbeitet man jeweils bei Temperaturen zwischen -20°C und +160°C, vorzugsweise zwischen 0°C und 140°C.

Die erfindungsgemäßen Verfahren (a), (b) und (c) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b) und (c) werden die jeweils benötigten Ausgangsstoffe

im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b) und (c) jeweils nach üblichen Methoden.

Die erfindungsgemäßen Wirkstoffe können als Defoliants, Desiccants, Krautabtötungsmittel, und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z.B. Erdölfraktionen, Alkohole, wie Butanol oder Glycol sowie deren Ester, Ketone, wie Aceton, Methylethylketon, Methylisobutylketon oder Cyclohexanon, stark polare Lösungsmittel, wie Dimethylformamid und Dimethylsulfoxid, sowie Wasser und mineralische oder pflanzliche Öle.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure,

Aluminiumoxid und Silikate sowie Ammoniumsalze; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azofarbstoffe, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Für die Mischungen kommen bekannte Herbizide wie zum Beispiel 1-Amino-6-ethylthio-3-(2,2-dimethylpropyl)-1,3,5-triazin-2,4-(1H, 3H)-dion oder N-(2-Benzthiazolyl)-N,N'-dimethyl-harnstoff zur Unkrautbekämpfung in Getreide, 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen in Frage. Einige dieser Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe konnen als solche, in Form ihrer Formulierungen oder den daraus durch weiteres Verdünnen bereiteten Anwendungsformen, wie gebrauchsfertige Lösungen, Suspensionen, Emulsionen, Pulver, und Granulate angewandt werden. Die Anwendung geschieht in üblicher Weise z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

## Herstellungsbeispiele

### Beispiel 1

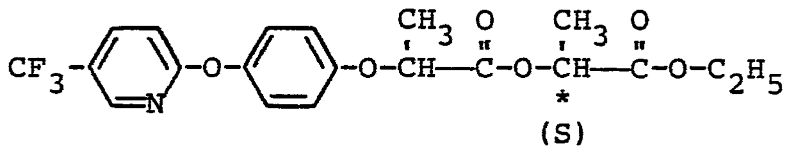

Eine Lösung von 6 g (0,0175 Mol) 2-/4-(5-Trifluormethyl-pyridyl-2-oxy)-phenoxy7-propionsäurechlorid in 50 ml Toluol wurde bei Temperaturen zwischen 0°C und 5°C in ein Gemisch aus 2,1 g (0,0175 Mol) S-Enantiomerem des Milchsäure-ethylesters und 1,9 g (0,019 Mol) Triethylamin in 50 ml Toluol gegeben. Man rührte weitere 14 Stunden bei Raumtemperatur und arbeitete dann auf, indem man das Reaktionsgemisch nacheinander mit verdünnter wäßriger Salzsäure und Wasser wusch, dann trocknete und durch Abziehen des Lösungsmittels unter vermindertem Druck einengte. Der verbleibende Rückstand wurde durch leichtes Erwärmen im Hochvakuum von Resten an flüchtigen Anteilen befreit. Man erhielt auf diese Weise 5 g (66,9 % der Theorie) an dem S-Enantiomeren des 2-/4-(5-Trifluormethyl-pyridyl-2-oxy)-phenoxy7-propionsäure-(1-ethoxycarbonyl)-ethylesters der oben angegebenen Formel.

Brechungsindex: $n_D^{21}$ = 1,5045

Drehwert: $[\alpha]_D^{24}$ = -5,4°

Beispiel 2

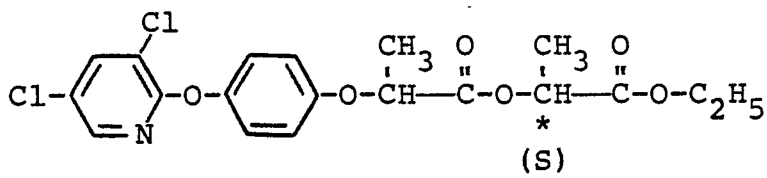

Nach der im Beispiel 1 angegebenen Methode wurde auch die Verbindung der obigen Formel erhalten.

Ausbeute: 56,1 % der Theorie

Brechungsindex: $n_D^{20,5} = 1,5449$

Drehwert: $[\alpha]_D^{24} = -6,9°$

In den folgenden Verwendungsbeispielen wurde die nachstehend angegebene Verbindung als Vergleichssubstanz eingesetzt:

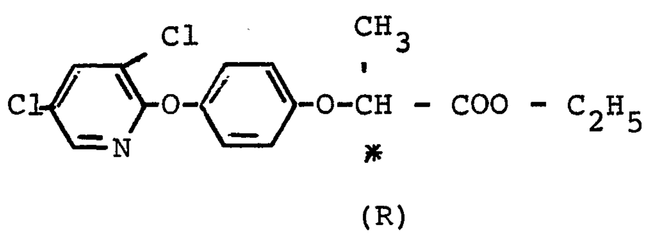

= (A)

Bekannt aus DE-OS 27 58 002

0126361

Beispiel A

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt die Verbindung gemäß Beispiel (2) bei der Bekämpfung von Avena fatua in Rüben eine bessere selektive herbizide Wirksamkeit als die Vergleichssubstanz (A).

0126361

Beispiel B

Post-emergence-Test / Gewächshaus

Lösungsmittel: 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2ooo l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle.
100 % = totale Vernichtung

In diesem Test zeigt die Verbindung gemäß Beispiel (2) eine sehr gute herbizide Wirksamkeit.

Patentansprüche

1) Optisch aktive Propionsäure-Derivate der Formel

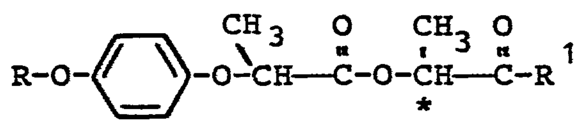

(I)

in welcher

R für die Reste der Formeln

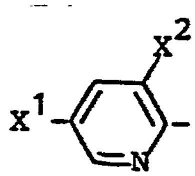

oder

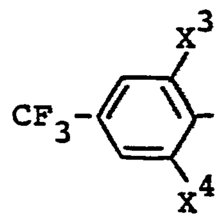

steht,

worin

$X^1$ für Halogen oder Trifluormethyl steht,

$X^2$ für Wasserstoff, Halogen oder Trifluormethyl steht,

$X^3$ für Wasserstoff oder Halogen steht und

$X^4$ für Wasserstoff oder Halogen steht,

und

$R^1$ für Alkoxy oder Alkylthio steht.

2) Optisch aktive Propionsäure-Derivate der Formel (I), in denen

R für die Reste

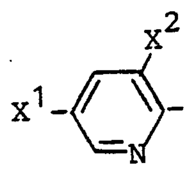 oder 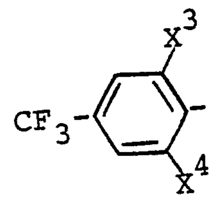 steht,

in welchen

$X^1$ für Chlor oder Trifluormethyl steht,

$X^2$ für Wasserstoff oder Chlor steht,

$X^3$ für Wasserstoff oder Chlor steht und

$X^4$ für Wasserstoff oder Chlor steht,

und

$R^1$ für Alkoxy mit 1 bis 4 Kohlenstoffatomen oder Alkylthio mit 1 bis 4 Kohlenstoffatomen steht.

3) Verfahren zur Herstellung von optisch aktiven Propionsäure-Derivaten der Formel

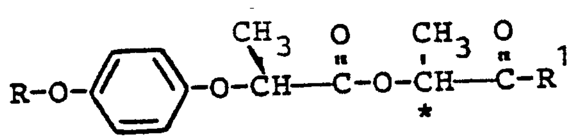 (I)

in welcher

R für die Reste der Formeln

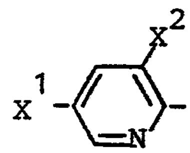 oder 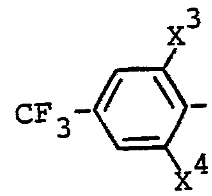 steht,

worin

$X^1$ für Halogen oder Trifluormethyl steht,

$X^2$ für Wasserstoff, Halogen oder Trifluormethyl steht,

$X^3$ für Wasserstoff oder Halogen steht und

$X^4$ für Wasserstoff oder Halogen steht,

und

$R^1$ für Alkoxy oder Alkylthio steht,

dadurch gekennzeichnet, daß man

a) Phenoxycarbonsäurechloride der Formel

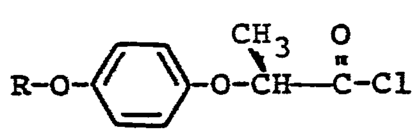 (II)

in welcher

R die oben angegebene Bedeutung hat,

mit optisch aktiven Milchsäure-Derivaten der Formel

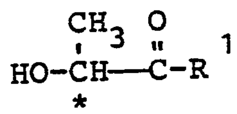

(III)

in welcher

$R^1$ die oben angegebene Bedeutung hat,

gegebenenfalls in Gegenwart eines Säureakzeptors sowie gegebenenfalls in Gegenwart eines Verdünnungsmittels umsetzt,

oder

b) Phenol-Derivate der Formel

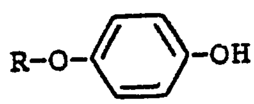

(IV)

in welcher

R die oben angegebene Bedeutung hat,

mit einem optisch aktiven Carbonsäure-Derivat der Formel

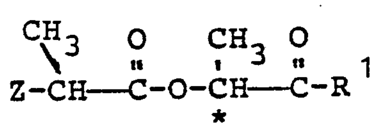

(V)

in welcher

$R^1$ die oben angegebene Bedeutung hat und

Z für Chlor, Brom, Tosylat oder Mesylat steht,

gegebenenfalls in Gegenwart eines Säureakzeptors sowie gegebenenfalls in Gegenwart eines Verdünnungsmittels umsetzt,

oder

c) Phenoxycarbonsäure-Salze der Formel

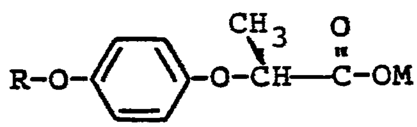

(VI)

in welcher

R die oben angegebene Bedeutung hat und

M für ein Alkalimetall-Kation oder für ein Äquivalent eines Erdalkalimetall-Kations steht,

mit optisch aktiven Halogenverbindungen der Formel

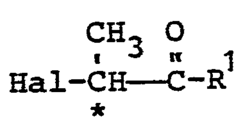

(VII)

in welcher

$R^1$ die oben angegebene Bedeutung hat und

Hal für Chlor oder Brom steht,

gegebenenfalls in Gegenwart eines Säureakzeptors sowie gegebenenfalls in Gegenwart eines Verdünnungsmittels umsetzt.

4) Herbizide Mittel, gekennzeichnet durch einen Gehalt an mindestens einem optisch aktiven Propionsäure-Derivat der Formel (I).

5) Verwendung von optisch aktiven Propionsäure-Derivaten der Formel (I) zur Bekämpfung von Unkraut.

6) Verfahren zur Bekämpfung von Unkraut, dadurch gekennzeichnet, daß man optisch aktive Propionsäure-Derivate der Formel (I) auf die Unkräuter und/oder deren Lebensraum ausbringt.

7) Verfahren zur Herstellung von herbiziden Mitteln, dadurch gekennzeichnet, daß man optisch aktive Propionsäure-Derivate der Formel (I) mit Streckmitteln und/oder oberflächenaktiven Stoffen vermischt.

8) Optisch aktives Propionsäure-Derivat der Formel

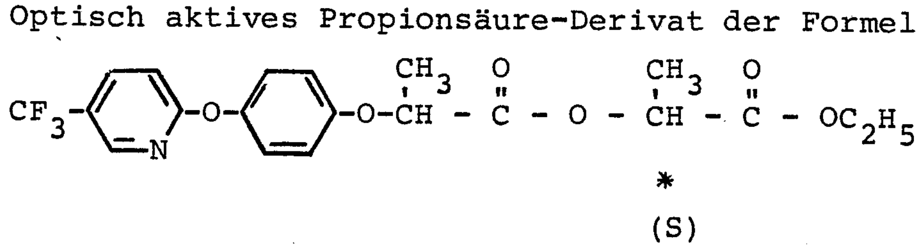

9) Optisch aktives Propionsäure-Derivat der Formel

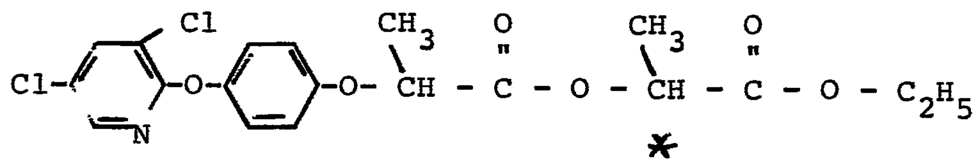

(S)

Europäisches Patentamt

**EUROPÄISCHER RECHERCHENBERICHT**

0126361

Nummer der Anmeldung

EP 84105088.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE - A1 - 2 732 846 (CIBA-GEIGY)<br>* Ansprüche 1,11,12,13 *<br>-- | 1,3b, 4,5 | C 07 D 213/64<br>C 07 C 69/712<br>C 07 C 67/14<br>C 07 C 67/10<br>C 07 C 67/00<br>C 07 C 67/31<br>A 01 N 43/40<br>A 01 N 37/38 |
| A | DE - A1 - 2 609 461 (HOECHST)<br>* Ansprüche 1-4 *<br>-- | 1,3b, 4,5 | |
| D,A | DE - A1 - 2 758 002 (HOECHST)<br>---- | | |
| | | | **RECHERCHIERTE SACHGEBIETE (Int. Cl. 3)** |
| | | | C 07 D 213/00<br>C 07 C 69/00<br>C 07 C 67/00 |

Der vorliegende Recherchenbericht wurde für alle Patentansprüche erstellt.

| Recherchenort | Abschlußdatum der Recherche | Prüfer |
|---|---|---|
| WIEN | 02-08-1984 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82